# EUROPEAN PATENT SPECIFICATION

(11) **EP 1 988 034 B1**
(45) Date of publication and mention of the grant of the patent: **20.10.2010**
(21) Application number: 07714369.1
(22) Date of filing: 16.02.2007
(51) Int. Cl.: B65D 83/00, B65D 33/01, B65D 33/25, B65D 81/26, B65D 85/00

(54) **CONTAINING BAG**
AUFNAHMEBEUTEL
SAC CONTENANT

(30) Priority: 24.02.2006 JP 2006047773
(43) Date of publication of application: 05.11.2008
(73) Proprietor: NS Planning Inc., Toshima-ku, Tokyo 1700005 (JP)
(72) Inventor: YOSHIDA, Tsukasa, Tokyo 170-0005 (JP)
(74) Representative: Gille Hrabal Struck Neidlein Prop Roos
(86) International application number: PCT/JP2007/052839
(87) International publication number: WO 2007/097255

(56) References cited:
- JP-A- 06 099 991
- JP-A- 08 085 578
- JP-U- 3 044 987
- US-A1- 2005 178 691

## Description

### TECHNICAL FIELD

The present invention relates to a containing bag in which a sheet or the like emitting fragrance can be contained and which can be housed in a pocket of clothes or a purse.

### BACKGROUND ART

Conventionally, as a containing bag in which the amount of fragrance or gas diffused from a contained matter can be regulated, there is according to the preamble of claim 1 a bag in which two rows of upper and lower zippers are provided to an aperture portion of the bag made of plastic films, holes are formed between the zippers, and the holes are covered with adhesive tapes. By opening or closing the zippers and sticking or peeling the adhesive tapes, the amount of diffusion of fragrance can be regulated (see Patent Document 1 below).
There is also a containing bag in which a small sheet is inserted into an opening and closing aperture of the bag to release gas emitted from a containedmatter (see Patent Document 2 below).

Patent Document 1: Japanese Patent Application Laid-open No. H6-99991
Patent Document 2: Japanese Utility Model Registration No. 3044987
US 2005/0178691 discloses a bag to facilitate the evacuation of excess air.

### DISCLOSURE OF THE INVENTION

### PROBLEM TO BE SOLVED BY THE INVENTION

A sheet impregnated or coated with perfume is contained in a containing bag and the containing bag is put into a pocket of clothes or a purse to be used like perfume in some cases.
In this case, if the containing bag described in Patent Document 1 is used, the adhesive tape is stuck on the openings to regulate the amount of diffusion, and therefore it is difficult to regulate the amount of diffusion of fragrance. Moreover, as the adhesive tape is repeatedly stuck on the openings, the adhesion weakens and therefore the adhesive tape may come unstuck and fall, and then lost.
When the containing bag described in Patent Document 2 is used, as in the above case, it is also difficult to regulate the amount of diffusion and the small sheet may be lost.

With the above problems in view, the present invention provides a containing bag in which the amount of fragrance or the like diffused from a contained matter can be regulated and there is no possibility of losing members for regulating the amount of diffusion.

### MEANS FOR SOLVING THE PROBLEM

A containing bag according to claim 1 is provided with a fastener portion capable of sealing a containing section at an opening aperture portion of a bag body having at least one containing section, and **characterized in that** a plurality of ventilation holding portions that can be inserted into the fastener portion are formed at the bag body and on an outer side of the fastener portion.
With this structure, by inserting the ventilation holding portions into the fastener portion and closing the fastener portion, it is possible to form vents to diffuse fragrance of a contained matter. Moreover, it is possible to regulate the amount of diffusion of fragrance by increasing or reducing the number of the ventilation holding portions to be inserted.

The ventilation holding portion may be formed in a concave-convex accordion shape along the fastener portion. With this structure, the concavity and convexity function as vents to further facilitate diffusion of fragrance of the contained matter.

Alternatively a containing bag according to claim 3 is provided with a fastener portion capable of sealing containing sections at an opening aperture portion of a bag body having at least two containing sections, and **characterized in that** a plurality of ventilation holding portions which can be inserted into the fastener portion are formed at a partition face portion to partition the containing sections and on an outer side of the fastener portion.
With this structure, the ventilation holding portions can be folded toward one face side or the other face side of the bag body and inserted into the fastener portions of both the containing sections to form vents for both the containing sections.

In the above containing bag, tear portions that can be torn from an outer end portion side of the bag body toward the fastener portion can be formed so that the ventilation holding portions can be formed by tearing the tear portions.
With this structure, the ventilation holding portions can be formed by tearing the tear portions, and thus, it is possible to form the necessary number of ventilation holding portions.

Alternatively a containing bag according to claim 5 is provided, wherein a fastener portion capable of sealing a containing section is provided at an upper end portion side of a sealed bag body having a substantially rectangular shape, an unsealing portion capable of unsealing the bag body in a width direction is formed on an upper side of the fastener portion, and a plurality of tear portions that can be torn from the upper end portion side toward the fastener portion are formed between the fastener portion and the unsealing portion at least on one face side of the bag body.
With this structure, it is possible to seal the bag before use and it is possible to unseal the bag at the unsealing portion and tear the tear lines so as to form the necessary number of ventilation holding portions at the time of use.

### BRIEF DESCRIPTION OF THE DRAWINGS

[FIG. 1] A schematic perspective view of a first embodiment of a containing bag of the present invention.
[FIG. 2] A partial enlarged sectional view showing a fastener portion of the containing bag in FIG. 1.
[FIG. 3] A schematic perspective view showing a state in which ventilation holding portions are inserted in the fastener portion of the containing bag in FIG. 1.
[FIG. 4] A partial enlarged view showing a modified example of the ventilation holding portions of the containing bag in FIG. 1.
[FIG. 5] Drawings, showing modified examples of the containing bag in FIG. 1, wherein (A) is a schematic perspective view and (B) shows a state in which tear lines are torn.
[FIG. 6] A schematic perspective view showing a second embodiment of the containing bag of the present invention.
[FIG. 7] A schematic front view showing a third embodiment of the containing bag of the present invention.

### BEST MODE FOR CARRYING OUT THE INVENTION

The present invention will be described below based on an embodiment shown in the drawings.

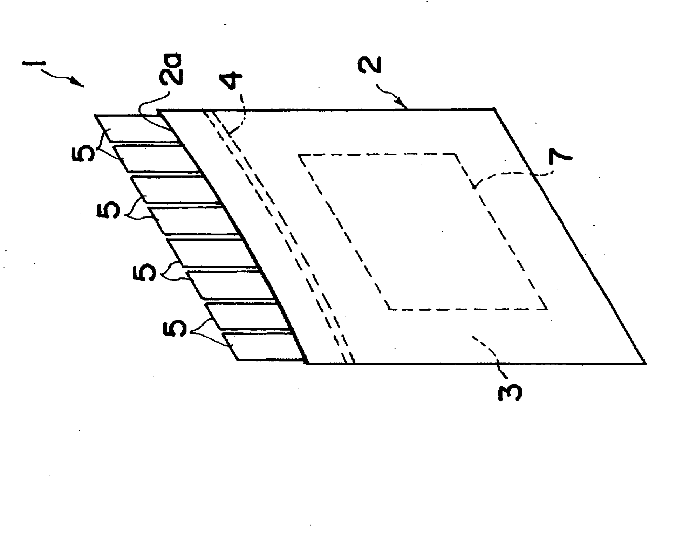

As shown in FIG. 1, in a containing bag 1 of a first embodiment of the present invention, an opening portion 2a is formed at an upper end portion of a bag body 2 having a substantially rectangular flat shape, the inside of the bag is used as a containing section 3, a fastener portion 4 parallel to the upper end portion is provided on the opening portion 2a side, and a plurality of ventilation holding portions 5, 5 ... are formed on one face side of the bag body 2 and above the fastener portion 4.

The bag body 2 is formed of a resin film. As the resin film, a film made of, for example, polyethylene, polypropylene, ethylene-vinyl acetate copolymer, polyester, or the like can be used. It is preferable to use a resin film having a gas barrier property. It is especially preferable to use an aluminum-evaporated or laminated film, e.g., a film formed by laminating polyester, aluminum, and polyethylene in this order from a surface side.

The bag body 2 is formed by folding one sheet of resin film to form a lower end portion and both side portions are heat-sealed. A method of sealing is not limited thereto, but sealing using a high frequency wave or an adhesive is also possible. Alternatively, two sheets of resin film, each having a substantially rectangular shape, may be overlapped with each other and three sides may be sealed.
Although the bag body 2 of the embodiment has the substantially rectangular flat shape, the shape is not limited thereto, but may be any shape such as a substantially elliptic shape or a slightly inflated shape.

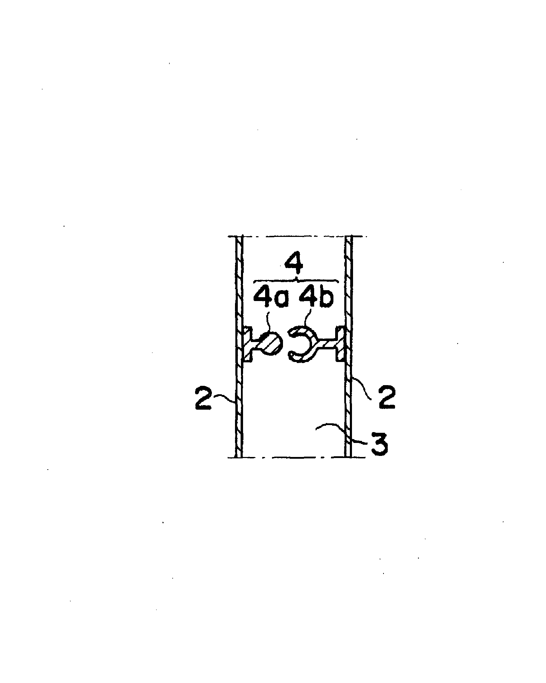

The fastener portion 4 is formed on inner faces of the bag body 2 and is capable of sealing the containing section 3 by opening and closing. As shown in FIG. 2, there is used a zipper in which a fitting portion 4a having a bulging tip end with a substantially circular section and a fitted portion 4b with a substantially C-shaped section to be fitted over the fitting portion 4a are disposed to face each other.
The fastener portion 4 not limited to the above structure but may be any structure that can seal the containing section 3 by fitting.

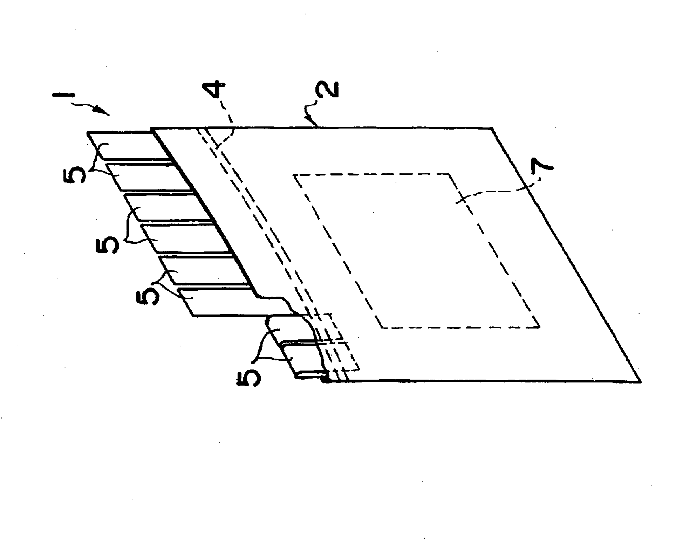

As shown in FIG. 1 or 3, the ventilation holding portions 5 are substantially rectangular strips that can be inserted between the fitting portion 4a and the fitted portion 4b of the fastener portion 4 and are formed on one face side (back face side in FIG. 3) of the bag body 2 along the fastener portion 4 by forming a plurality of substantially-vertical slits from the upper end portion side. Moreover, the upper end portion of the one face side is formed to be higher than an upper end portion of the other face side to make it easy to pinch the ventilation holding portions 5.
Although eight ventilation holding portions 5 are provided in the embodiment, the number of the portions 5 is not limited thereto, but any number of ventilation holding portions can be formed.

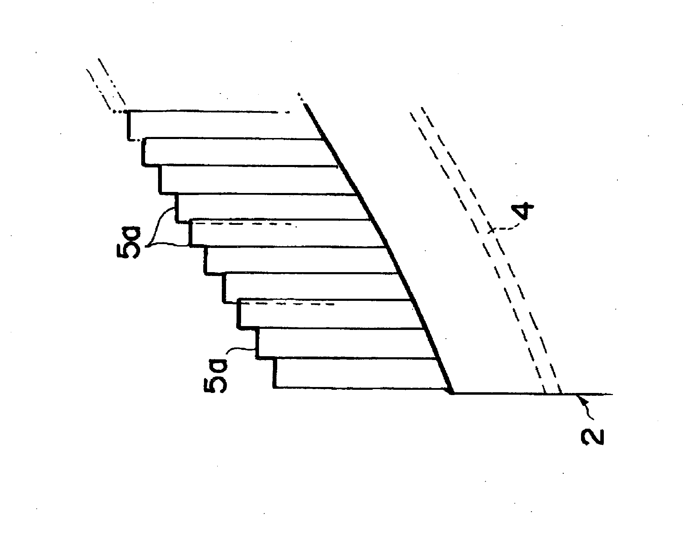

The ventilation holding portions 5 can be formed as an accordion ventilation holding portion 5a in which ridges and grooves extending in a vertical direction are alternately formed as shown in FIG. 4. By forming the concave-convex accordion ventilation holding portion 5a along the fastener portion 4 in this manner, vents can be easily formed.
The shape of the ventilation holding portion 5 may be the substantially rectangular shape or any other shapes such as a shape having a semicircular tip end.
Although the ventilation holding portions 5 are provided on the one face side in the embodiment, they may be provided on both face sides. Furthermore, although the ventilation holding portions 5 are provided throughout the width of the upper end portion, the ventilation holding portions 5 may be provided only at a part of the upper end portion.

Usage of the containing bag 1 of one embodiment of the present invention will be described below.
The fastener portion 4 of the containing bag 1 is opened and a sheet 7 or the like emitting fragrance is contained in the containing section 3 as shown in FIG. 1. Next, as shown in FIG. 3, the ventilation holding portions 5 are folded so that tip end portions are inserted into the containing section 3, and positioned between the fitting portion 4a and the fitted portion 4b of the fastener portion 4. In this state, the fitting portion 4a and the fitted portion 4b are fitted with each other to close the fastener portion 4. Because the portion where the ventilation holding portions 5 are inserted cannot be closed, it functions as a vent to diffuse the fragrance emitted from the sheet. The containing bag 1 may be put into an inside pocket of a suit, a purse, or the like and used like perfume.
It is possible to increase the amount of diffusion of fragrance by increasing the number of the ventilation holding portions 5 to be inserted. On the other hand, it is possible to reduce the amount of diffusion of fragrance by reducing the number of ventilation holding portions 5 to be inserted. In this way, the containing bag 1 of the present invention can regulate the amount of diffusion of fragrance. Moreover, because the ventilation holding portions 5 are formed at the bag body 2, there is no possibility of losing them.
In order to prevent emission of fragrance, the ventilation holding portions 5 may be pulled out of the fastener portion 4 and the fastener portion 4 may be closed to seal the containing section 3.
A sheet formed by kneading perfume of rose, lavender, or the like into cyclodextrin made from corn starch or the like and applying it onto a sheet of paper or impregnating the sheet of paper with it may be used as the sheet that emits fragrance.

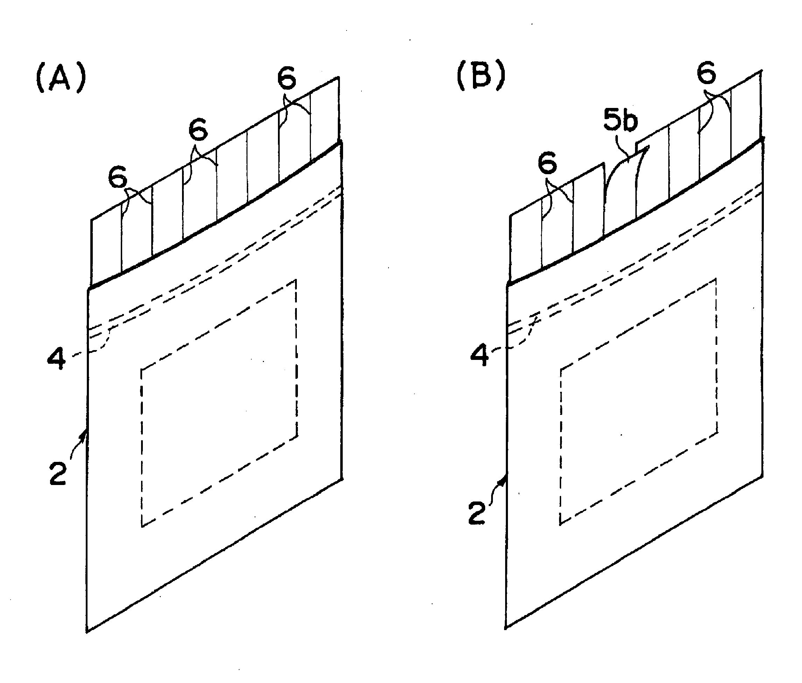

As a modified example of the containing bag 1 of the embodiment, streaky tear lines 6 may be formed substantially vertically from the upper end portion side of the bag body 3 as shown in FIG. 5(A) and ventilation holding portions 5b may be formed by tearing the tear lines 6 as shown in FIG. 5(B). The tear lines 6 may be formed as streaks that are thinner than the other parts, may be formed by making holes of dotted lines, or may be formed by stretching the film to facilitate tearing of the film in the vertical direction.

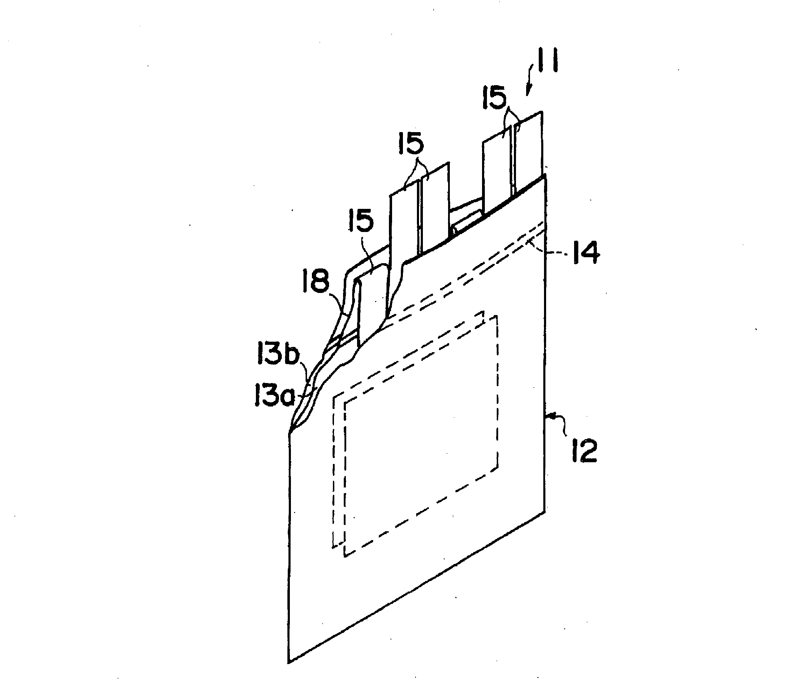

Another embodiment of the containing bag of the present invention will be described below. In the description, description of portions having the same structures as those in the first embodiment will be omitted.
The second embodiment of the containing bag of the present invention is a containing bag 11 having two containing sections 13a, 13b on the front and back of a bag body 12 as shown in FIG. 6. Ventilation holding portions 15 are formed at a partition face portion 18 to partition the containing sections 13a, 13b. By folding the ventilation holdingportions 15 forward or backward and inserting them into a fastener portion 14, the vents can be formed at both the containing sections 13a, 13b.
Although the ventilation holding portions 15 are formed at the partition face portion 18 in the embodiment, they are not necessarily formed there but may be provided on a front face or a back face of the bag body. Moreover, three or more containing sections may be formed.

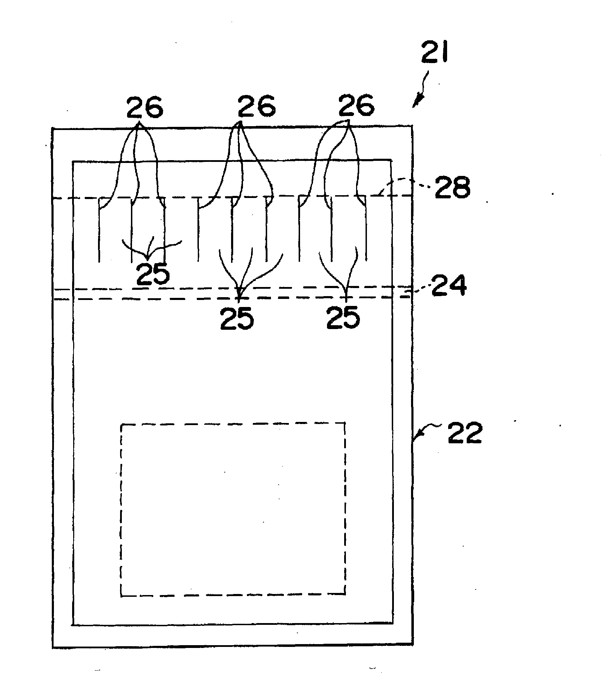

In a containing bag of a third embodiment of the present invention, as shown in FIG. 7, four sides of a bag body 22 are sealed by heat sealing or the like in advance, an unsealing portion 28 where the bag body 22 can be unsealed in a width direction and a fastener portion 24 are formed on an upper end portion side of the bag body 22, and a plurality of streaky tear lines 26 extending from the upper end portion side toward the fastener portion 24 are formed on one face side (front side in FIG. 7) of the bag body 22.
The unsealing portion 28 can be formed as a streak that is thinner than the other parts or may be formed by stretching the resin films to facilitate unsealing in the width direction.
The unsealing portion 28 may be torn in the width direction and the tear lines 26 may be torn from the upper end portion side toward the fastener portion 4 to form the ventilation holding portions 25.
Because the sheet or the like emitting fragrance can be sealed in the containing bag 21 in advance, the fragrance of the enclosed sheet is prevented from escaping before use.

## Claims

1. A containing bag (1) provided with a fastener portion (4) capable of sealing a containing section (3) at an opening aperture portion (2a) of a bag body (2) having at least one containing section (3), **characterized by**
a plurality of ventilation holding portions (5) that can be inserted into the fastener portion (4) are formed at the bag body (2) and on an outer side of the fastener portion (4).

2. The containing bag (1) according to claim 1, wherein
the ventilation holding portion (5), is formed in a concave-convex accordion shape along the fastener portion (4).

3. A containing bag (11) provided with a fastener portion (14) capable of sealing containing sections (13) at an opening aperture portion (12a) of a bag body (12) having at least two containing sections (13a,13b), **characterized by**
a plurality of ventilation holding portions (15) which can be inserted into the fastener portion (14) are formed at a partition face portion to partition the containing sections (13a,13b) and on an outer side of the fastener portion (14).

4. The containing bag according to any one of claims 1 to 3, wherein
tear portions (6) that can be torn from an outer end portion side of the bag body (2) toward the fastener portion (4) are formed so that the ventilation holding portions (5) can be formed by tearing the tear portions (6).

5. A containing bag (21) provided with a fastener portion (24) capable of sealing a containing section at an opening aperture portion of a bag body (22) and a containing section, **characterized in that** the fastener portion (24) is provided at an upper end portion side of a sealed bag body (22) having a substantially rectangular shape, an unsealing portion (28) capable of unsealing the bag body (22) in a width direction is formed on an upper side of the fastener portion (24), and a plurality of tear portions (26) that can be torn from the upper end portion side toward the fastener portion (24) to form the ventilation holding portions (25) are formed between the fastener portion (24) and the unsealing portion (28) at least on one face side of the bag body (22).

## Patentansprüche

1. Eine Aufnahmetasche 1, die mit einem Befestigungsbereich 4 versehen ist, der einen Aufnahmebereich 3 in einem zu öffnenden Bereich 2a mit einem Durchlass einer Taschenstruktur 2 mit mindestens einem Aufnahmebereich 3 abdichten kann, **dadurch gekennzeichnet, dass**
eine Vielzahl von Belüftungsaufnahmebereichen 5, die in den Befestigungsbereich 4 eingeführt werden können, in der Taschenstruktur 2 und auf einer Außenseite des Befestigungsbereichs 4 ausgebildet werden.

2. Die Aufnahmetasche 1 gemäß Anspruch 1, wobei
der Belüftungsaufnahmebereich 5 in einer konkav-konvexen Akkordeonform entlang dem Befestigungsbereich 4 ausgebildet wird.

3. Eine Aufnahmetasche 11, die mit einem Befestigungsbereich 14 versehen ist, der die Aufnahmebereiche 13 in einem zu öffnenden Bereich 12a mit Durchlass einer Taschenstruktur 12 mit mindestens zwei Aufnahmebereichen 13a, 13b versiegeln kann, **dadurch gekennzeichnet, dass**
eine Vielzahl von Belüftungsaufnahmebereichen 15, die in den Befestigungsbereich 14 eingeführt werden können, in einer Unterteilungswand für die Unterteilung der Aufnahmebereiche 13a, 13b und auf einer Außenseite des Befestigungsbereichs 14 ausgebildet werden.

4. Die Aufnahmetasche gemäß einem der Ansprüche 1 bis 3, wobei
Abreißbereiche 6, die vom äußeren Endabschnitt der Taschenstruktur 2 in Richtung des Befestigungsbereichs 4 abgerissen werden können, so geformt werden, dass die Belüftungsaufnahmebereiche 5 durch Abreißen der Abreißbereiche 6 geformt werden können.

5. Eine Aufnahmetasche 21, die mit einem Befestigungsbereich 24 versehen ist, der einen Öffnungsbereich einer Taschenstruktur 22 ermöglicht, und einen Aufnahmebereich aufweist, **dadurch gekennzeichnet, dass** der Befestigungsbereich 24 am oberen Abschnitt einer abgedichteten Taschenstruktur 22 angeordnet ist, welche eine im Wesentlichen rechteckige Form aufweist, dass ein Bereich 28 zum Trennen der Versiegelung, mit dem die Versiegelung der Taschenstruktur 22 in Querrichtung getrennt werden kann, auf der Oberseite des Befestigungsbereichs 24 ausgebildet ist, sowie dass eine Vielzahl von Abreißbereichen 26, die vom oberen Abschnitt in Richtung des Befestigungsbereichs 24 abgerissen werden können, um die Belüftungsaufnahmebereiche 25 auszubilden, zwischen dem Befestigungsbereich 24 und dem Bereich 28 zum Trennen der Versiegelung mindestens auf einer Seite der Taschenstruktur 22 ausgebildet werden.

## Revendications

1. Sac de rangement (1) muni d'un corps de sac (2) comportant au moins une portion de rangement (3) et une portion d'attache (4) qui est apte à fermer hermétiquemet ladite portion de rangement (3) dans l'ouverture (2a) dudit corps de sac, **caractérisé en ce que**
une pluralité de portions de maintien de ventilation (5) qui peuvent être insérées dans la portion d'attache (4) sont formées sur le corps de sac (2) et sur le côté extérieur de la portion d'attache (4).

2. Sac de rangement (1) selon la revendication 1, dans lequel une portion de maintien de ventilation (5) se présentent sous forme de dépliant (en zigzag ou en accordéon) au long de la portion d'attache (4).

3. Sac de rangement (11) muni d'un corps de sac (12) comportant au moins deux portions de rangement (13a, 13b) et une portion d'attache (14) qui est apte à fermer hermétiquemet lesdites portions de rangement (13) dans l'ouverture (12a) dudit corps de sac, **caractérisé en ce que**
une pluralité de portions de maintien de ventilation (15) qui peuvent être insérées dans la portion d'attache (14) sont formées sur une face de cloison qui sépare les portions de rangement (13a, 13b) et sur le côté extérieur de la portion d'attache (14).

4. Sac de rangement selon la revendication 1 à 3,
dans lequel il est prévu des portions à déchirer ou trancher (6) depuis la partie extrémité du corps de sac (2) vers la portion d'attache (4), afin de former les portions de maintien de ventilation (5) en faisant une incision dans ladite portion (6) .

5. Sac de rangement (21), equipé d'une portion d'attache (24) qui est apte à fermer hermétiquement une portion de rangement dans l'ouverture d'un corps de sac (22), **caractérisé en ce que** la portion d'attache (24) est formée sur l'extrémité supérieure du corps de sac (22) qui est fermé et qui est sensiblement rectangulaire, ce sac comportant en outre une portion à décacheter (28) sur la partie supérieure de la portion d'attache (24) pour ouvrir le corps de sac (22) dans une direction transversale, et une plurarité de portions à déchirer ou trancher (26) depuis la portion supérieure vers la portion d'attache (24) entre la portion d'attache (24) et la portion à décacheter (28), au moins sur une face du corps de sac (22), pour ainsi réaliser les portions de maintien de ventilation (25).
